(19)

Europäisches Patentamt
European Patent Office
Office européen des brevets

(11) Publication number: **0 555 086 B1**

# (12) EUROPEAN PATENT SPECIFICATION

(45) Date of publication of patent specification: **27.12.95**

(51) Int. Cl.6: **A61K 7/08**

(21) Application number: **93300837.7**

(22) Date of filing: **04.02.93**

The file contains technical information submitted after the application was filed and not included in this specification

## (54) Cosmetic composition

(30) Priority: **07.02.92 GB 9202568**

(43) Date of publication of application:
**11.08.93 Bulletin 93/32**

(45) Publication of the grant of the patent:
**27.12.95 Bulletin 95/52**

(84) Designated Contracting States:
**AT BE CH DE DK ES FR GB GR IE IT LI NL PT SE**

(56) References cited:
**EP-A- 0 070 077**
**EP-A- 0 358 216**
**EP-A- 0 409 005**

(73) Proprietor: **UNILEVER PLC**
**Unilever House**
**Blackfriars**
**London EC4P 4BO (GB)**
(84) Designated Contracting States:
**GB IE**

(73) Proprietor: **UNILEVER N.V.**
**Weena 455**
**NL-3013 AL Rotterdam (NL)**
(84) Designated Contracting States:
**BE CH DE DK ES FR GR IT LI NL PT SE AT**

(72) Inventor: **Shana'a, May**
**17 Gardens Road,**
**Bebington**
**Wirral L63 7OZ (GB)**
Inventor: **Steer, David Charles**
**"Thornwood",**
**Tower Road North,**
**Heswall**
**Wirral L60 6RS (GB)**

(74) Representative: **Bristow, Stephen Robert et al**
**Unilever PLC**
**Patent Division**
**Colworth House**
**Sharnbrook**
**Bedford MK44 1LO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to cosmetic compositions and in particular to liquid, aqueous shampoo compositions containing a combination of surfactants for use on the body or the hair.

Shampoo compositions may be formulated to select those components, especially surfactant ingredients, which together result in compositions which possess one or more, preferably a combination, of the following beneficial properties:

(a) the shampoo compositions are stable; that is, the components of the composition do not suffer from chemical instability (decomposition) and/or physical instability (sedimentation/creaming/setting), and in particular the components of a shampoo composition should not be incompatible with each other;

(b) the shampoo compositions possess adequate foaming capacity, especially the ability to generate foam quickly, easily and foam which is rich and creamy;

(c) in the case of hair shampoos, the compositions are capable of providing one or more conditioning benefits such as smoothness, softness, gloss, anti-static properties and ease of combing thereof, or at least do not negatively affect such attributes;

(d) in the case of body shampoos in particular, but also in the case of shampoos for the hair, the compositions are mild to the skin and do not promote dryness or flakiness thereof;

(e) the shampoo compositions look attractive, and for example may be optically clear or translucent;

(f) the components of the composition should be derived from natural sources and be readily biodegradable, thereby rendering the product "environmentally friendly".

Alkyl polyglycosides (APG's) are known for inclusion in body and hair shampoo compositions and have the advantages that they are mild surface active agents, biodegradable and obtainable from renewable raw materials. However, APG's give only moderate foam volumes and the foam is slow to generate. The foam quality is also poor, due to lack of richness/creaminess, and frequently sensory properties such as smoothness and gloss of hair and skin treated with APG's are inferior.

EP-A-0409005 describes a low-irritant, good-foaming shampoo comprising an APG, a sucrose fatty acid ester surfactant and an anionic and/or amphoteric surfactant among which soaps are mentioned. it is described how that use of a large amount of alkyl saccharide surfactants produces coarse and non-creamy foam when incorporated in a shampoo or body shampoo which requires creamy foam.

GB-A-2139243 describes the use of APG's in conjunction with anionic polymers where it is alleged that the anionic polymer function is to improve the detergency of the APG's and mildness of their foam.

GB-A-976088 describes APG's per se and mentions their incorporation into soap bars for the purpose of enhancing the dispersibility of the soap, as well as for increasing the detergency and biodegradability of the soap product.

EP-A-0070074 and EP-A-0070075 disclose foaming composition comprising a mixture of surfactants, including an APG and an anionic cosurfactant among which soaps are mentioned.

EP-A-0463912 describes toilet soap bars containing APG to achieve improved foaming, lather, skin feel and moisturisation, lime dispersion and cleaning.

US-A-4396520 describes solid detergent granules comprising APG, calcium-sensitive anionic detergent, e.g. soap, and preferably a detergent builder.

We have now surprisingly found that in the context of aqueous hair and skin shampoo compositions comprising an APG as the principal surfactant component, by incorporation also of a particular kind of fatty acid soap having a short chain length, preferably at a low level relative to the APG, it is possible to produce a shampoo composition having significantly improved foaming characteristics, whilst retaining an overall better balance of other properties, in particular mildness to skin and hair and also beneficial sensory properties such as smoothness and, in the case of hair, other conditioning attributes. This overall optimized balance of foaming and other properties has hitherto not been achieved or even addressed in the prior art referred to above.

Accordingly, in one aspect the present invention provides a liquid, aqueous shampoo composition, for example a hair shampoo, body shampoo, shower gel or the like, comprising:

(a) as a principal surfactant component, at least one alkyl polyglycoside; and

(b) at least one short chain fatty acid soap having a carbon chain length of up to about $C_{16}$, the weight ratio of short chain soap to alkyl polyglycoside being within the range of from 1:2 to 1:20.

The invention, and in particular preferred embodiments and aspects thereof, will now be described in detail.

Alkyl polyglycoside

The alkyl polyglycoside (APG) may be any APG which is capable of being employed in cosmetic compositions. Typically, the APG is one which comprises an alkyl group connected (optionally via a bridging group) to a block of one or more glycosyl groups. Preferred APG's are described by the following formula:

$RO\text{-}(G)_n$

wherein R is a branched or straight chain alkyl group which may be saturated or unsaturated and G is a saccharide group.

R may represent a mean alkyl chain length of from about $C_5$ to about $C_{20}$. Preferably, R represents a mean alkyl chain length of from about $C_9$ to about $C_{12}$. Most preferably the value of R lies between about 9.5 and about 10.5. G may be selected from $C_5$ or $C_6$ monosaccharide residues or mixtures of $C_5$ and $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. More preferably the value of n is up to about 1.5. Most preferably the value of n lies in the range of from about 1.3 to about 1.5.

Preferred APG's for use in the invention are those which contain no or substantially no alkoxylate groups in the chain, as the presence of such groups renders the APG less readily biodegradable.

Suitable alkyl polyglycosides for use in the invention are commercially available and include for example those materials identified as: Oramix NS10 ex Seppic; and APG225, APG300 (corresponding to the material sold as PLANTAREN 2000), APG350, APG550 and APG600 (alternatively known as PLANTAREN 1200) ex Henkel.

The level of APG as the principal surfactant component in the shampoo compositions of the invention is preferably from about 1 to about 40% wt, more preferably from about 5 to about 30% wt, most preferably from about 10 to about 20% wt.

Short chain soap

The short chain soap may be any short chain soap which enhances the foam volume, speed of lathering and/or foam quality of the APG. Preferred short chain soaps are those which are soluble in the shampoo composition.

The short chain soap is a soap which has a carbon chain length of up to about $C_{16}$, more preferably up to about $C_{12}$ or $C_{14}$. The short chain soap may be a linear or branched chain soap and may be saturated or unsaturated. Most preferably the short chain soap is a saturated, linear short chain soap.

The most preferred short chain soap length, particularly for compositions for use on the hair, is from about $C_8$ to about $C_{12}$ or $C_{14}$, especially $C_8$ to $C_{10}$ or $C_{12}$. Suitable short chain length soaps for use in the present invention include those of caprylic, capric, myristic and lauric acids and mixtures thereof.

$C_8$ to $C_{10}$ soaps are especially good, e.g. compared with $C_{12}$ to $C_{16}$ soaps, for giving enhanced foam volume as well as density and also, in the context of hair shampoos, for avoiding negative effects on attributes such as gloss.

In general we have found that shorter chain length soaps are preferable over longer chain length soaps in terms of an overall enhanced foaming, measured for example by parameters such as rate of generation of foam, amount and creaminess of foam (both visual and tactile perceptions), ease of rinse, slipperiness of foam covered and rinse hair, coarse/dry feel and ease of wet combing.

The amount of short chain soap present in the shampoo compositions of the invention may be selected so as to give the desired degree of foam quality/quantity enhancement. Preferably the short chain soap is present in an amount of from about 1 to about 20% wt, more preferably from about 1 to about 12% wt, even more preferably from about 2 to about 6% wt.

In preferred embodiments, in the compositions of the invention the short chain soap is present in a relatively low amount compared with the APG, such that the soap:APG weight ratio is most preferably from about 1:3 to 1:10. Generally enough of the soap component should be present in order to achieve, in conjunction with the APG surfactant component, the beneficial combination of properties characteristic of the compositions of the invention.

Other ingredients

Other components which may or may not be present in compositions of the instant invention include thickeners, for example naturally occurring polysaccharides or derivatives thereof such as starch, xanthan gums, carrageenan gum and the like, alginates such as sodium alginate, naturally occurring soluble starches, synthetic polymers, e.g. cellulose gums and other cellulose derivatives such as hydroxypropyl cellulose, polyacrylates, either in combination with naturally occurring soluble viscosity agents or alone, clays and salts such as sodium chloride, ammonium chloride.

Suitable viscosity agents may be present in a range of from about 0.01% by weight to about 4% by weight. Preferably, a viscosity agent, if present, makes up from about 0.2% by weight to about 1% by weight of the compositions.

Other optional components of the compositions of the present invention include waxes such as castor wax, candelilla wax, carnuba wax and beeswax, monoglycerides, sucrose esters, sorbitan esters and the like.

A further optional component of the compositions in accordance with the invention is a cationic conditioning polymer, e.g. a cationic derivative of guar gum. Suitable cationic guar gum derivatives are those given the CTFA designation guar hydroxypropyl trimonium chloride, available commercially for example as JAGUAR C13S. Other suitable derivatives include those designated JAGUAR C15, C17, C16, and 162. The cationic polymer may be present in an amount of from about 0.05% to about 0.5% by weight.

Further optional components of the compositions of the present invention include one or more additional surfactants for example selected from anionic, nonionic, amphoteric and zwitterionic surfactants, conditioning agents, buffering agents, oils such as those derived from plants or other natural sources, e.g. clove oil, and other components commonly employed in shampoo compositions, such as perfumes, colourings, emollients, suspending agents, stabilisers, opacifiers, pearlisers, preservatives and buffering agents.

In general, it is preferred that any optional components present in the compositions of the invention do not adversely affect the naturally derived nature, biodegradability and beneficial range of physical and sensory properties which are characteristic of this invention.

In a further aspect of the present invention, a process for producing a shampoo composition as hereinbefore described includes adding a basic solution, typically a solution of sodium hydroxide, to the short chain fatty acid dispersed in water. After mixing, this solution may be added to the APG with rapid mixing. Alternatively, a solution of the APG may be premixed with the short chain fatty acid at a temperature above the melting point of the acid, and subsequently neutralised with the solution of sodium hydroxide. When a viscosity agent is used, it may be added to an aqueous NaOH solution with vigorous mixing at high shear. After the viscosity agent has dispersed providing an evenly mixed solution free from lumps, liquid fatty acid may then be added.

Should the fatty acid be solid at ambient temperature it may be melted before addition to the basic solution. APG may then be added to the soap solution with rapid mixing.

An opacifier may also be employed in the process of the present invention, but this is not mandatory. Should an opacifier be included in the composition, it may be heated and mixed together with the APG to a temperature of the order of about 80 °C. Suitable opacifiers include behenyl alcohol and castor wax. Once the opacifier melts and disperses in the APG, the mixture may be added to the basic solution with rapid mixing.

The invention will now be further illustrated with reference to the following examples. It is to be understood that the examples are not intended to limit the scope of the invention. All amounts are in % wt, unless otherwise stated.

Examples

Examples 1 to 6 (Table 1) illustrate compositions of the present invention in the form of shampoos for the hair. The compositions of Examples 7 to 14 (Table 2) are suitable for use as body shampoos/shower gels.

TABLE 1

| Ingredient | Example | | | | | |
|---|---|---|---|---|---|---|
| | 1 | 2 | 3 | 4 | 5 | 6 |
| Oramix NS10[1] | 12 | - | 13 | 12 | 15 | 13 |
| APG600[2] | - | 12 | - | - | - | - |
| Caprylic acid | 4 | 5 | - | - | - | - |
| Capric acid | - | - | 4 | 5 | - | - |
| Coconut fatty acid | - | - | - | - | 3 | 4 |
| Nacol 22-97[3] | - | - | - | - | - | 0.5 |
| Castorwax | - | - | 0.4 | 0.4 | - | - |
| Sucrose laurate | - | - | - | - | 1 | - |
| Rilanit GMRO[4] | - | - | 0.4 | - | 0.4 | - |
| Montanol 68[5] | - | - | - | 1.00 | - | 2.0 |
| Beeswax | - | - | - | - | 0.4 | - |
| Klucel M[6] | 0.8 | - | - | - | - | - |
| Trisodium citrate[7] | 1.0 | 1.0 | 1.0 | 1.0 | 1.0 | 1.0 |
| Sodium chloride | - | 1.0 | 5.0 | 2.5 | 3.0 | 2.0 |
| 5% NaOH | -----------to pH7------- | | | | -to pH7.5- | |
| Perfumes, preservatives colouring etc. | qs | qs | qs | qs | qs | qs |
| Water | ----------------to 100---------------- | | | | | |

1) $C_{10}$ alkyl glucoside, DP 1.3-1.4
2) $C_{12-14}$ alkyl glucoside, DP 1.4
3) Behenyl alcohol
4) Glycerol monoricinoleate
5) Glucoside wax
6) Hydroxypropyl cellulose
7) Buffering agent

TABLE 2

| Ingredient | Example | | | | | | | |
|---|---|---|---|---|---|---|---|---|
| | 7 | 8 | 9 | 10 | 11 | 12 | 13 | 14 |
| APG600 | 16 | 15 | 14 | - | - | - | - | 9.5 |
| Oramix NS10 | - | - | - | 15 | 14 | 16 | 16 | 5.5 |
| Coconut fatty acid | 4 | 5 | 6 | 5 | 6 | 4 | 4 | 5 |
| Clay - Laponite | 1 | 1 | 4 | 1 | 1 | 1 | - | 1 |
| Bentone | - | - | - | - | - | - | 2 | - |
| Perfume, preservative colouring etc. | qs | qs | qs | qs | qs | qs | qs | qs |
| NaOH | to pH8 | | | | | | | |
| Water | to 100 | | | | | | | |

Comparative Examples 1 and 2

The following Comparative Tests 1 and 2 were performed between a control formulation containing no short chain soap, compositions A and B which contained short chain soaps and composition C which contained a long chain soap, as depicted in the following table:-

| Ingredient | Control | Composition (wt%) | | |
|---|---|---|---|---|
| | | A | B | C |
| Oramix NS10 | 18 | 16 | 16 | 16 |
| Caprylic acid | - | 2.0 | - | - |
| Capric acid | - | - | 2.0 | - |
| Oleic acid | - | - | - | 2.2 |
| 5% NaOH solution | to pH7 | | | to pH8 |
| Water | to 100 | | | |

Comparative Test 1 - Arm wash test

Panellists were asked to generate foam in an arm wash test wherein measured amounts of shampoo had been applied to their hands for lathering prior to rubbing the foam on the inside of the forearm. 12 panellists were used in paired comparison tests between two 6-membered groups. The first group used one product first while the second group used the other product first. The paired test was also balanced between the use of left and right forearms. The panellists were then asked to score each product for various descriptors by magnitude estimation.

Results

| | Control vs A | Control vs B | Control vs C |
|---|---|---|---|
| Ease of foaming | A | B | C |
| Amount of foam | A | B | C |
| Creaminess of foam | A | B | C |

The product indicated in each case was preferred (≧95% significance). Thus, for the first three descriptors the product indicated had a higher score (easier foaming, more foam, creamier foam)

Conclusion - all three types of soap gave greatly improved lather quantity and quality, compared with the use of no soap at all in combination with the APG.

Comparative Test 2 - Test of dry hair properties

This is a paired comparison test in which 3 hair switches were washed with one product and 3 with the other product. 12 panellists were used. The switches were assessed in pairs and the panellists were asked to indicate which of the two switches they preferred (left or right) for various attributes.

Results

| | Control vs A | Control vs B | Control vs C |
|---|---|---|---|
| Gloss | n.s | n.s | control |
| Smoothness | n.s | n.s | control |
| Ease of comb | n.s | n.s | control |
| Lack of flyaway | A | n.s | control |

The product indicated in each case was preferred (≧95% significance, n.s. - no significant difference).

Conclusion

A and B (caprylic and capric soaps, respectively) give superior dry hair properties as compared to C (oleic soap). A and B show no negatives on dry hair properties, whereas C does.

Comparative Example 3

This Example demonstrates the effect of carbon chain length of the short chain soap on lathering properties and product stability of a skin shampoo composition.

The following three shampoos D, E and F were prepared with the following compositions:

| Ingredient | Composition (wt%) | | |
|---|---|---|---|
| | D | E | F |
| Oramix NS10 | 10 | 10 | 10 |
| APG600 | 5 | 5 | 5 |
| Capric ($C_{10}$) soap | 5 | - | - |
| Lauric ($C_{12}$) soap | - | 5 | - |
| Oleic ($C_{18}$) soap | - | - | 5 |
| Laponite | 1 | 1 | 1 |
| Perfume, preservative, etc | qs | qs | qs |
| NaOH | to pH8.5 | to pH8.5 | to pH8.5 |
| Water | to 100 | to 100 | to 100 |

Each of the above shampoos was subjected to an arm wash test in which the foam volume and creaminess were measured. Each of 20 trained panellists wearing surgical gloves firstly wetted their hands and then dispensed thereon 0.5g of product which was then worked into a lather.

Lather volume and creaminess were measured using magnitude estimations at 4 second intervals, the results given below being normalised average figures for each product. The actual lather volume for each product was also measured quantitatively. The results are given in the table below.

| | Composition | | |
|---|---|---|---|
| | D | E | F |
| Foam volume | 1.13 | 0.94 | 0.5 |
| Foam creaminess | 1.00 | 0.94 | 0.6 |
| Measured foam volume (ml) | 72 | 62 | 50 |

To test stability, each composition was allowed to stand for 3 months at 37°C. Shampoos D and E remained clear, whereas shampoo F showed precipitation of solid soap crystals, indicating that the product had destabilised.

Comparative Example 4

This Example demonstrates the effect of short chain soap:APG weight ratio (with a $C_{12}$ soap) on lather properties and mildness of a skin shampoo composition.

The following three shampoos G, K and I were prepared with the following compositions:

| Ingredient | Composition (wt%) | | |
|---|---|---|---|
| | G | H | I |
| Oramix NS10 | 12 | 10 | 10 |
| APG600 | 4 | 5 | 3 |
| $C_{12}$ soap | 4 | 5 | 6 |
| Laponite | 1 | 1 | 1 |
| Perfume, preservation, etc | qs | qs | qs |
| NaOH | to pH8 | to pH8 | to pH8 |
| Water | to 100 | to 100 | to 100 |

Each of the above shampoos was subjected to the following tests:

Test I - Arm-wash test

20 trained panellists conducted an arm-wash test by firstly pre-wetting the forearm and then dispensing directly into the hand 0.5g of the test product which was then lathered on the forearm for 10 seconds. The arm was rinsed under running water until fully rinsed and then pat-dried with a paper towel. During and after (after a 2 minute wait) the wash procedure each panellist was questioned as to whether they thought each product tested satisfied the following criteria : product disperses easily; product lathers easily; product gives copious lather; product gives creamy lather. The results in the table below are expressed as a percentage of the panellists who thought each criterion was satisfied.

| Results | Composition | | |
|---|---|---|---|
| | G | H | I |
| Disperses easily | 78.9 | 77.3 | 77.1 |
| Lathers easily | 64.0 | 70.4 | 73.2 |
| Copious lather | 60.4 | 69.5 | 66.2 |
| Creamy lather | 65.8 | 70.8 | 71.6 |

Test II - Zein test

Each composition was tested for mildness on the skin in accordance with the Zein test, the protocol of which is described for example in (i) E. Gott : Proceedings of the 4th International Congress of Surface Active Substances, Brussels 1964, volume 3, pages 83-90, or (ii) M. J. Schwinger : Kolloid - Z.Z. Poly., 1969, volume 233, page 898, the disclosures of both of which references are incorporated herein by reference.

The following results were obtained, the lower the Zein value indicating a milder product:

| Composition | Zein value (%N) |
|---|---|
| G | 0.04 |
| H | 0.06 |
| I | 0.14 |

## Claims

**1.** A liquid, aqueous shampoo composition suitable for application to skin or hair, comprising:
(a) as a principal surfactant component, at least one alkyl polyglycoside; and
(b) at least one short chain fatty acid soap having a carbon chain length of up to $C_{16}$, the weight ratio of short chain to alkyl polyglycoside being within the range of from 1:2 to 1:20.

**2.** A shampoo composition according to claim 1, wherein the alkyl polyglycoside has the formula:

$$RO\text{-}(G)_n$$

wherein R is a branched or straight chain alkyl group G is a saccharide group, and n is the degree of polymerisation.

**3.** A shampoo composition according to claim 2, wherein R has a mean alkyl chain length of from $C_5$ to $C_{20}$.

**4.** A shampoo composition according to claim 2 or claim 3, wherein n lies in the range 1 to 10.

**5.** A shampoo composition according to claim 4, wherein the value n is up to 1.5.

**6.** A shampoo composition according to any one of claims 2 to 5, wherein G is selected from $C_5$ or $C_6$ monosaccharide residues or mixtures of $C_5$ and $C_6$ monosaccharide residues.

**7.** A shampoo composition according to any preceding claim, wherein the APG is present in an amount of from 1 to 40% by weight.

8. A shampoo composition according to any preceding claim, wherein the short chain soap length is from $C_8$ to $C_{14}$.

9. A shampoo composition according to claim 8, wherein the short chain soap length is from $C_8$ to $C_{12}$.

10. A shampoo composition according to claim 8, wherein the short chain soap is selected from soaps of caprylic, capric, myristic and lauric acids and mixtures thereof.

11. A shampoo composition according to any preceding claim, wherein the short chain soap is present in an amount of from 1 to 20% by weight.

12. A shampoo composition according to any preceding claim, further comprising one or more additional components selected from surfactant, conditioning agents, buffering agent, opacifier, thickening agent, cationic conditioning polymer and wax.

13. A shampoo composition according to any preceding claim, which is a hair shampoo.

14. A shampoo composition according to any preceding claims, which is a body shampoo or shower gel.

15. A method of washing hair or skin, comprising applying thereto a shampoo composition according to any preceding claim.

**Patentansprüche**

1. Flüssige, wäßrige Shampoozusammensetzung geeignet für das Auftragen auf Haut oder Haare, umfassend:
   (a) als eine hauptsächliche oberflächenaktive Komponente mindestens ein Alkylpolyglycosid; und
   (b) mindestens eine kurzkettige Fettsäure-Seife mit einer Kohlenstoffkettenlänge von bis zu $C_{16}$, wobei das Gewichtsverhältnis von kurzkettiger Seife zu Alkylpolyglycosid in dem Bereich von 1:2 bis 1:20 liegt.

2. Shampoozusammensetzung gemäß Anspruch 1, worin das Alkylpolyglycosid die Formel:

   $RO\text{-}(G)_n$

   hat, worin R eine verzweigt- oder geradkettige Alkylgruppe ist, G eine Saccharidgruppe ist und n der Grad der Polymerisation ist.

3. Shampoozusammensetzung gemäß Anspruch 2, worin R eine mittlere Alkylkettenlänge von $C_5$ bis $C_{20}$ hat.

4. Shampoozusammensetzung gemäß Anspruch 2 oder Anspruch 3, worin n in dem Bereich von 1 bis 10 liegt.

5. Shampoozusammensetzung gemaß Anspruch 4, worin der Wert von n bis zu 1,5 beträgt.

6. Shampoozusammenstzung gemäß einem der Ansprüche 2 bis 5, worin G ausgewählt ist aus $C_5$- oder $C_6$- Monosaccharidresten oder Mischungen aus $C_5$- und $C_6$- Monosaccharidresten.

7. Shampoozusammensetzung gemäß einem der vorhergehenden Ansprüche, worin das APG in einer Menge von 1 bis 40 Gewichts-% vorliegt.

8. Shampoozusammensetzung gemäß einem der vorhergehenden Ansprüche, worin die Länge der kurzkettigen Seife von $C_8$ bis $C_{14}$ reicht.

9. Shampoozusammensetzung gemäß Anspruch 8, worin die Länge der kurzkettigen Seife von $C_8$ bis $C_{12}$ reicht.

**10.** Shampoozusammensetzung gemäß Anspruch 8, worin die kurzkettige Seife ausgewählt ist aus Seifen der Capryl-, Caprin-, Myristin- und Laurinsäure und Mischungen davon.

**11.** Shampoozusammensetzung gemäß einem der vorhergehenden Ansprüche, worin die kurzkettige Seife in einer Menge von 1 bis 20 Gewichts-% vorliegt.

**12.** Shampoozusammensetzung gemäß einem der vorhergehenden Ansprüche, weiterhin umfassend eine oder mehrere zusätzliche Komponenten ausgewählt aus einem oberflächenaktiven Stoff, Konditioniermitteln, Puffermittel, Trübungsmittel, Verdickungsmittel, kationischem Konditionierpolymer und Wachs.

**13.** Shampoozusammensetzung gemäß einem der vorhergehenden Ansprüche, die ein Haarshampoo ist.

**14.** Shampoozusammensetzung gemäß einem der vorhergehenden Ansprüche, die ein Körpershampoo oder Duschgel ist.

**15.** Verfahren Haare oder Haut zu waschen, umfassend das Darauf-Auftragen einer Shampoozusammensetzung gemäß einem der vorhergehenden Ansprüchen.

**Revendications**

**1.** Composition de shampooing liquide aqueuse appropriée pour l'application à la peau et aux cheveux, qui comprend :

(a) à titre de composant tensioactif principal, au moins un polyglycoside alkylique ; et

(b) au moins un savon d'acide gras à chaîne courte ayant une longueur de chaîne carbone jusqu'à $C_{16}$, le rapport pondéral de la chaîne courte au polyglycoside alkylique étant dans la gamme de 1:2 à 1:20.

**2.** Composition de shampooing selon la revendication 1, dans laquelle le polyglycoside alkylique répond à la formule :

$RO-(G)_n$

dans laquelle R est un radical alkyle à chaîne droite ou ramifiée, G est un groupe saccharide et n est le degré de polymérisation.

**3.** Composition de shampooing selon la revendication 2, dans laquelle R a une longueur de chaîne alkyle moyenne de $C_5$ à $C_{20}$.

**4.** Composition de shampooing selon la revendication 2 ou 3, dans lequel n se situe entre 1 et 10.

**5.** Composition de shampooing selon la revendication 4, dans laquelle la valeur de n est jusqu'à 1,5.

**6.** Composition de shampooing selon l'une quelconque des revendications 2 à 5, dans laquelle G est choisi parmi des restes de monosaccharide en $C_5$ ou $C_6$ ou des mélanges de restes de monosaccharide en $C_5$ et $C_6$.

**7.** Composition de shampooing selon l'une quelconque des revendications précédentes, dans laquelle le PGA est présent à raison de 1 à 40% en poids.

**8.** Composition de shampooing selon l'une quelconque des revendications précédentes, dans laquelle la longueur de chaîne du savon à chaîne courte est de $C_8$ à $C_{14}$.

**9.** Composition de shampooing selon la revendication 8, dans laquelle la longueur de chaîne du savon à chaîne courte est de $C_9$ à $C_{12}$.

**10.** Composition de shampooing selon la revendication 8, dans laquelle le savon à chaîne courte est choisi parmi les savons d'acides caprylique, caprique, myristique et laurique et leurs mélanges.

**11.** Composition de shampooing selon l'une quelconque des revendications précédentes, dans laquelle le savon à chaîne courte est présent à raison de 1 à 20% en poids.

**12.** Composition de shampooing selon l'une quelconque des revendications précédentes, qui comprend en outre un ou plusieurs composants supplémentaires choisis parmi les tensioactifs, les agents de conditionnement, les agents tampons, les opacifiants, les agents épaississants, les polymères de conditionnement cationiques et les cires.

**13.** Composition de shampooing selon l'une quelconque des revendications précédentes, qui est un shampooing pour les cheveux.

**14.** Composition de shampooing selon l'une quelconque des revendications précédentes, qui est un shampooing corporel ou un gel de douche.

**15.** Procédé de lavage des cheveux ou de la peau, qui consiste à leur appliquer une composition de shampooing selon l'une quelconque des revendications précédentes.